# EUROPÄISCHE PATENTANMELDUNG

(11) **EP 0 943 685 A2**
(43) Veröffentlichungstag der Anmeldung: **22.09.1999**
(21) Anmeldenummer: 99101164.4
(22) Anmeldetag: 22.01.1999
(51) Int. Cl.: C12N 15/12, C07K 14/705, C07K 16/28, A61K 48/00, C12Q 1/68, G01N 33/576, G01N 33/68, C12N 5/10

(54) **Neuer G-Protein-gekoppelter Rezeptor aus menschlichem Gehirn**

(30) Priorität: 11.02.1998 DE 19805351
(71) Anmelder: BASF AKTIENGESELLSCHAFT, 67056 Ludwigshafen (DE)
(72) Erfinder: Kröger, Burkhard, Dr., 67117 Limburgerhof (DE); Otterbach, Bernd, Dr., 67061 Ludwigshafen (DE)

(57) **Zusammenfassung**

Neuer G-Protein gekoppelter Rezeptor aus menschlichem Gehirn, das dafür codierendes Gen und seine Verwendung

## Beschreibung

Die vorliegende Erfindung betrifft einen neuen G-Protein-gekoppelten Rezeptor aus humanem Gehirn, dessen Gene und Verwendung.

G-Protein-gekoppelte Rezeptoren stellen eine Superfamilie integraler Membranproteine dar. Familienmitglieder sind Rezeptoren für alle Typen chemischer Botenstoffe, aber auch Sensoren für Licht und Geruch. G-Protein-gekoppelte Rezeptoren kommen in fast allen Organismen vor.

Die Rezeptoren sind charakterisiert durch eine Aminosäuresequenz mit 7 ausgeprägt hydrophoben Bereichen. Diese stellen höchstwahrscheinlich memoranständige Domänen dar und geben der Familie ihren zweiten Namen: 7-Transmembran-Domänen Rezeptoren.

Die Liganden dieser Rezeptorfamilie sind mit biogenen Aminen (z.B. Adrenalin, Serotonin, Histamin), Peptidhormonen (z.B. Angiotensin, Endothelin, Bradykinin), Neurotransmittern (z.B. NPY, Substanz P, Opioide) und Proteinen (z.B. Chemokine, Thrombin) sehr vielfältig. Alle diese Messenger sind durch Interaktion mit G-Proteinen an der Signalübertragung in das Innere der Zelle beteiligt. Als Effektoren sind bekannt: Adenylatcyclase, Phospholipase C, Phosphodiesterase.

Die G-Protein-gekoppelten Rezeptoren werden in drei Unterfamilien unterteilt: die Rhodopsin-Unterfamilie, Calcitonin-Unterfamilie und Glutamat/metabotrope-Unterfamilie.

Gegenstand der Erfindung ist ein isoliertes Protein, enthaltend die in SEQ ID NO:2 dargestellte Aminosäuresequenz oder eine daraus durch Substitution, Insertion oder Deletion von einem oder mehreren Aminosäureresten erhältliche Sequenz, wobei wenigstens noch eine der wesentlichen biologischen Eigenschaften des in SEQ ID NO:2 dargestellten Proteins erhalten bleibt.

Die wesentliche biologische Eigenschaft ist in der Aktivität als Rezeptor, insbesondere als G-Protein gekoppelter Rezeptor zu sehen.

Ein weiterer Gegenstand der Erfindung sind Proteine mit G-Protein gekoppelter Rezeptoraktivität, die ausgehend von der in SEQ ID NO:2 dargestellten Aminosäuresequenz durch gezielte Veränderungen herstellbar sind. Beispielsweise können bestimmte Aminosäuren durch solche mit ähnlichen physikochemischen Eigenschaften (Raumerfüllung, Basizität) ersetzt werden. Es können aber auch ein oder mehrere Aminosäuren hinzugefügt oder entfernt werden, oder mehrere dieser Maßnahmen miteinander kombiniert werden. Die solchermaßen gegenüber der SEQ ID NO:2 veränderten Proteine besitzen wenigstens 60 %, bevorzugt wenigstens 75 % Homologie zu SEQ ID NO: 2, berechnet nach dem Algorithmus von Pearson und Lipman, Proc. Natl. Acad. Sci (USA) 85, 2444-2448.

Ein weiterer Gegenstand der Erfindung sind Nukleinsäuresequenzen, die für die o.g. Proteine codieren, insbesondere solche mit der in SEQ ID NO:1 dargestellten Primärstruktur.

Die vorliegende cDNA kann durch dem Fachmann geläufige Klonierungs- und Transfektionsmethoden in verschiedenen Expressionssystemen zur Expression gebracht werden. Dies sind beispielsweise pro- oder eukaryotische Vektorsysteme, wie SV40, CMV, Baculovirus, Adenovirus; Plasmide; Phagemide, Phagen.

Dazu wird die erfindungsgemäße Nukleinsäuresequenz üblicherweise mit genetischen Regulationselementen wie Transkriptions- und Translationssignalen funktionell verknüpft. Mit den solchermaßen hergestellten rekombinanten Nukleinsäurekonstrukten werden anschließend Wirtsorganismen transformiert.

Eine bevorzugte Ausführungsform ist die Verknüpfung der erfindungsgemäßen Nukleinsäuresequenz mit einem Promotor, wobei der Promotor 5' upstream zu liegen kommt. Weitere Regulationssignale wie Terminatoren, Polyadenylierungssignale, Enhancer können in dem Nukleinsäurekonstrukt Anwendung finden.

Als Wirtszellen sind Bakterien wie Escherichia coli, eukaryotische Mikroorganismen wie Saccharomyces cerevisiae, höhere eukaryotische Zellen aus Tieren oder Pflanzen, beispielsweise Sf9 oder CHO-Zellen, geeignet.

Gewünschtenfalls kann das Genprodukt auch in transgenen Organismen wie transgenen Tieren, z.B. Mäusen, Schafen oder transgenen Pflanzen zur Expression gebracht werden.

Bei der vorliegenden Erfindung handelt es sich um eine cDNA, die für ein neues Mitglied der G-Protein-gekoppelten Rezeptorfamilie kodiert. Sequenzvergleiche zeigen Verwandtschaft mit Endothelinrezeptoren und Endothelinrezeptor-ähnlichen Sequenzen.

Die vorliegende Nukleotidsequenz wurde ursprünglich in mehreren cDNA-Bibliotheken aus humanem Gehirn und Rückenmark identifiziert. Die Analyse der Verteilung der zugehörigen mRNA in 50 verschiedenen menschlichen Geweben ergab fast ausschließliche Expression im Gehirn.

Das durch die vorliegende cDNA kodierte Polypeptid läßt sich zweifelsfrei als G-Protein-gekoppelter Rezeptor identifizieren. Die 7 Transmembran-Domänen, das Kennzeichen dieser Proteinfamilie, lassen sich leicht im Hydrophilizitäts-Plot (Kyte, J. and R.F. Doolittle, 1982, J. Mol. Biol. 157, 105-132 (1982)) finden. Die größte Verwandtschaft auf Aminosäureebene findet sich mit 52 % Identität zu einem putativen Endothelinrezeptor Typ B-ähnlichen Protein humanen Ursprungs (Genbank Accession Nummer U87460, (FastA-Programm, Pearson und Lipman, Proc. Natl. Acad. Sci (USA) 85, 2444-2448).

In besonderen Fällen kann das Genprodukt auch in transgenen Tieren, z.B. Mäusen, Schafen oder transgenen Pflanzen zur Expression gebracht werden. Ebenso ist es möglich, zellfreie Translationssysteme mit der entsprechenden RNA zu programmieren.

Darüberhinaus kann das Genprodukt auch in Form therapeutisch oder diagnostisch geeigneter Fragmente exprimiert werden. Zur Isolation des rekombinanten Proteins können Vektorsysteme oder Oligonukleotide verwendet werden, die die cDNA um bestimmte Nukleotidsequenzen verlängern und damit für veränderte Polypeptide kodieren, die einer einfacheren Reinigung dienen. Als solche "Tags" sind in der Literatur z.B. Hexa-Histidin-Anker bekannt oder Epitope, die als Antigene verschiedener Antikörper erkannt werden können (beschrieben zum Beispiel in Harlow, E. and Lane, D. (1988) Antibodies: A Laboratory Manual. Cold Spring Harbor (N.Y.) Press).

Ausgehend von der Peptidsequenz können synthetische Peptide generiert werden, die als Antigene für die Produktion von Antikörpern eingesetzt werden. Es ist auch möglich, das Polypeptid oder Bruchstücke davon zur Generierung von Antikörpern einzusetzen.

Die Herstellung von Antikörpern ist eine dem Fachmann geläufige Tätigkeit. Mit Antikörpern sind sowohl polyklonale, monoklonale, humane oder humanisierte Antikörper oder Fragmente davon, single chain Antikörper oder auch synthetische Antikörper gemeint.

Die vorliegende cDNA bietet außerdem die Voraussetzung, die genomische Sequenz dieses neuen G-Protein-gekoppelten Rezeptors zu klonieren. Darunter fällt auch die dazugehörige regulatorische oder Promotorsequenz, die beispielsweise durch Sequenzierung des 5' upstream Bereiches der vorliegenden cDNA zugänglich wird. Die Sequenzinformation der cDNA ist auch die Grundlage für die Herstellung von antisense Molekülen oder Ribozymen.

Eine weitere Möglichkeit des Einsatzes der Nukleotidsequenz oder Teilen davon ist die Erzeugung transgener Tiere. Transgene Überexpression oder genetischer Knockout der Sequenzinformation in geeigneten Tiermodellen kann wertvolle weitere Informationen über die (Patho-)Physiologie des neuen G-Protein-gekoppelten Rezeptors.

In Situationen, in denen ein Mangel an dem beschriebenen Rezeptor (Protein gemäß Anspruch 1) herrscht, können mehrere Methoden zur Substituierung eingesetzt werden. Zum einen kann das Protein, natürlich oder rekombinant direkt, oder durch geeignete Maßnahmen in Form seiner kodierenden Nukleinsäure (DNA oder RNA) appliziert werden. Dazu können sowohl virale, als auch nichtvirale Vehikel zum Einsatz kommen.

Ein weiterer Weg bietet sich durch die Stimulation des endogenen, körpereigenen Genes durch geeignete Mittel. Auch der turn-over oder die Inaktivierung z.B. durch Rezeptorkinasen können blockiert werden. Schließlich können Agonisten dieses Rezeptors zum Einsatz gelangen.

In Situationen, in denen überschüssiger Rezeptor vorliegt, können verschiedene Inhibitoren eingesetzt werden. Diese Inhibition kann sowohl durch antisense Moleküle oder Ribozyme, oder Antikörper und Oligonukleotide, als auch durch niedermolekulare Verbindungen erreicht werden. Darüberhinaus kann der Rezeptor auch durch Antagonisten blockiert werden.

Weiterhin können die cDNA, die genomische DNA, der Promotor, als auch das Polypeptid, sowie Teilfragmente davon in rekombinanter oder nichtrekombinanter Form zur Ausarbeitung eines Testsystems verwendet werden. Dieses Testsystem ist geeignet, die Aktivität des Promotors oder des Proteins in Anwesenheit einer Testsubstanz zu messen. Bevorzugt handelt es sich dabei um einfache Meßmethoden (colorimetrischer, luminometrischer, fluorimetrischer, immunologischer oder radioaktiver Art,) die die schnelle Meßbarkeit einer Vielzahl von Testsubstanzen erlauben. Die beschriebenen Testsysteme erlauben die Bindung oder Agonisierung oder Antagonisierung von Testsubstanzen in Bezug zum neuen Rezeptor zu beschreiben.

Die Bestimmung von Menge, Aktivität und Verteilung des Rezeptors oder seiner zugrundeliegenden mRNA im menschlichen Körper kann zur Diagnose, Prädisposition und zum Monitoring bei bestimmten Erkrankungen dienen. Desgleichen kann die Sequenz der cDNA sowie der genomischen Sequenz zu Aussagen über genetische Ursachen und Prädispositionen bestimmter Erkrankungen herangezogen werden. Dazu können sowohl DNA/RNA-Proben, sowie unnatürliche DNA/RNA-Proben, als auch Antikörper verschiedenster Art benutzt werden. Dabei dient die beschriebene Nukleotidsequenz oder Teile davon in Form geeigneter Proben zur Aufdeckung von Punktmutationen oder Deletionen/Insertionen.

Weiterhin kann das beschriebene Protein benutzt werden, um seine natürlichen Liganden zu bestimmen und zu isolieren. So kann der Rezeptor rekombinant in Zellkultur exprimiert und sein Aktivierungszustand z.B. anhand des cAMP-Spiegels abgeleitet werden. Der cAMP-Spiegel läßt sich immunologisch oder mittels Reportertechnologie ermitteln. Damit ergibt sich auch ein diagnostisches Verfahren den Spiegel des Rezeptorliganden in Körperflüssigkeiten oder Geweben zu bestimmen.

Die erfindungsgemäße Nukleinsäuresequenz und das von ihr kodierte Protein können zur Entwicklung von Reagenzien, Agonisten und Antagonisten zur Diagnose und Therapie von chronischen und akuten Erkrankungen des zentralen und peripheren Nervensystems, wie Alzheimer's, Depression, Demenz, Motilitätsstörungen, Hirntumore, Schmerz, Schizophrenie, Angstzustände, Schlaganfall, Schlafstörungen, Apnoen, Husten, Psychosen, Parkinson's, Epilepsie, ALS, Drogenabhängigkeit, Lähmungen, sowie zur Cerebroprotektion und bei Erkrankungen mit nervöser Komponente, wie Obesity, Anorexie, Bulimie eingesetzt werden.

Ein weiterer Gegenstand der Erfindung ist Verfahren zum qualitativen und quantitativen Nachweis einer Nukleinsäure nach Anspruch 3 in einer biologischen Probe, das folgende Schritte umfaßt:
a) Inkubation einer biologischen Probe mit einer bekannten Menge an Nukleinsäure gemäß Anspruch 3 oder einer bekannten Menge an Oligonukleotiden, die als Primer für eine Amplifikation der Nukleinsäure gemäß Anspruch 3 geeignet sind,
b) Nachweis der Nukleinsäure gemäß Anspruch 3 durch spezifische Hybridisierung oder PCR-Amplifikation,
c) Vergleich der Menge an hybridisierender Nukleinsäure gemäß Anspruch 3 oder an durch PCR Amplifikation gewonnener Nukleinsäure gemäß Anspruch 3 mit einem Standard.

Weiterhin umfaßt die Erfindung ein Verfahren zum qualitativen und quantitativen Nachweis eines Proteins gem‰ß Anspruch 1 in einer biologischen Probe, das folgende Schritte umfaßt:
a) Inkubation einer biologischen Probe mit einem Antikörper, der spezifisch gegen das Protein gemäß Anspruch 1 gerichtet ist,
d) Nachweis des Antikörper/Antigenkomplexes,
c) Vergleich der Mengen des Antikörper/Antigenkomplexes mit einem Standard.

Als Standard können biologische Proben wie Gewebestücke, Serum oder Blut dienen, die von gesunden Probanden entnommen wurden.

### Beispiel 1

### Klonierung der Rezeptor cDNA

Bei der Sequenzanalyse von cDNA-Klonen einer cDNA-Bibliothek aus menschlichem Gehirn wurde zunächst eine Teilsequenz identifiziert. Die Sequenz dieses Teilklones umfaßt den Bereich zwischen Nukleotidposition 352 und 2411 in SEQ ID NO:1.

Aus einer cDNA-Bibliothek aus menschlichem Gehirn (Human Brain 5'Stretch Plus cDNA Library, # HL5018t, Fa. Clontech, Jahr 1997) wurde die Gesamtsequenz SEQ ID NO:1 mittels geschachtelter Polymerase Chain Reaktion amplifiziert. Dazu kamen folgende Oligonukleotidprimer zur Anwendung:
PCR1: mit SEQ ID NO: 3 und SEQ ID NO: 4;
PCR2: mit SEQ ID NO: 3 und SEQ ID NO: 5.

### Beispiel 2

### Expression des neuen Rezeptors in menschlichen Geweben

Die Expression des neuen Rezeptors wurde in 50 verschiedenen menschlichen Geweben mittels RNA-Dotblot-Analyse untersucht. Ein Blot der Firma Clontech (#7770-1) wurde dazu mit einer Rezeptor-Probe hybridisiert. Die Probe wurde durch *in vitro* Transkription der entsprechenden cDNA in Anwesenheit Digoxigenin-markierter Nukleotide hergestellt. Nach stringentem Waschen wurde das Transkript hauptsächlich in Gehirngewebe nachgewiesen.

## Patentansprüche

1. Isoliertes Protein, enthaltend die in SEQ ID NO:2 dargestellte Aminosäuresequenz oder eine daraus durch Substitution, Insertion oder Deletion von einem oder mehreren Aminosäureresten erhältliche Sequenz, wobei wenigstens noch eine der wesentlichen biologischen Eigenschaften des in SEQ ID NO:2 dargestellten Proteins erhalten bleibt.

2. Protein nach Anspruch 1, dadurch gekennzeichnet, daß es sich um ein humanes Protein handelt.

3. Nukleinsäuresequenz codierend für ein Protein nach Anspruch 1.

4. Nukleinsäuresequenz nach Anspruch 3, dadurch gekennzeichnet, daß sie für ein Protein codiert, das wenigstens 60 % Identität mit der in SEQ ID NO:2 dargestellten Sequenz hat.

5. Nukleinsäuresequenz nach Anspruch 4, dadurch gekennzeichnet, daß sie die in SEQ ID NO:1 dargestellte Sequenz enthält.

6. Rekombinantes Nukleinsäurekonstrukt, enthaltend eine Nukleinsäuresequenz gemäß Anspruch 3 funktionell verknüpft mit mindestens einem genetischen Regulationselement.

7. Wirtsorganismus, transformiert mit einer Nukleinsäuresequenz nach Anspruch 3.

8. Wirtsorganismus, transformiert mit einem rekombinanten Nukleinsäurekonstrukt nach Anspruch 6.

9. Verwendung eines Proteins nach Anspruch 1 als Antigen zur Erzeugung von spezifischen Antikörpern.

10. Antikörper, die spezifisch das Protein nach Anspruch 1 erkennen.

11. Verwendung einer Nukleinsäuresequenz zur Gentherapie.

12. Verwendung einer zu der Sequenz gemäß Anspruch 3 komplementären Nukleinsäuresequenz zur Gentherapie.

13. Verfahren zur Identifizierung von Antagonisten und Agonisten für das Protein gemäß Anspruch 1 indem man Zellen, die das Protein gemäß Anspruch 1 auf der Zelloberfläche tragen mit einer Vielzahl zu untersuchender Substanzen (Testsubstanzen) zusammenbringt, und anschließend die biologische Aktivität des Rezeptors in An- und Abwesenheit der Testsubstanz vergleicht.

14. Verfahren zum Testen von Substanzen hinsichtlich ihrer Eigenschaft als Ligand für das Protein gemäß Anspruch 1 zu fungieren, das folgende Schritte umfaßt:
a) Expression des Proteins nach Anspruch 1 in eukaryontischen Zellen,
b) Inkubation dieser Zellen mit Proteinextrakten, bevorzugt aus Gehirngewebe stammend,
c) Ermittlung der Bindung der zu untersuchenden Substanz an das Protein gemäß Anspruch 1 und und der Aktivierung durch Messung der cAMP Konzentration oder des Calciumflusses in der Zelle.

15. Verfahren zum qualitativen und quantitativen Nachweis einer Nukleinsäure nach Anspruch 3 in einer biologischen Probe, das folgende Schritte umfaßt:
a) Inkubation einer biologischen Probe mit einer bekannten Menge an Nukleinsäure gemäß Anspruch 3 oder einer bekannten Menge an Oligonukleotiden, die als Primer für eine Amplifikation der Nukleinsäure gemäß Anspruch 3 geeignet sind,
b) Nachweis der Nukleinsäure gemäß Anspruch 3 durch spezifische Hybridisierung oder PCR-Amplifikation,
c) Vergleich der Menge an hybridisierender Nukleinsäure gemäß Anspruch 3 oder an durch PCR Amplifikation gewonnener Nukleinsäure gemäß Anspruch 3 mit einem Standard.

16. Verfahren zum qualitativen und quantitativen Nachweis eines Proteins gemäß Anspruch 1 in einer biologischen Probe, das folgende Schritte umfaßt:
a) Inkubation einer biologischen Probe mit einem Antikörper, der spezifisch gegen das Protein gemäß Anspruch 1 gerichtet ist,
b) Nachweis des Antikörper/Antigenkomplexes,
c) Vergleich der Mengen des Antikörper/Antigenkomplexes mit einem Standard.


SEQUENCE LISTING

<110> BASF Aktiengesellschaft

<120> Neuer G-Protein gekoppelter Rezeptor aus menschlichem
      Hirn

<130> OZ 0050/48774

<140>
<141>

<150> DE 19805351.7
<151> 1998-02-11

<160> 5

<170> PatentIn Ver. 2.0

<210> 1
<211> 2411
<212> DNA
<213> Homo sapiens

<220>
<221> 5'UTR
<222> (1)..(19)

<220>
<221> CDS
<222> (20)..(1462)

<220>
<221> 3'UTR
<222> (1463)..(2411)

<400> 1
gtctcctgct catccagcc atg cgg tgg ctg tgg ccc ctg gct gtc tct ctt   52
                     Met Arg Trp Leu Trp Pro Leu Ala Val Ser Leu
                       1               5                  10

gct gtg att ttg gct gtg ggg cta agc agg gtc tct ggg ggt gcc ccc   100
Ala Val Ile Leu Ala Val Gly Leu Ser Arg Val Ser Gly Gly Ala Pro
             15                  20                  25

ctg cac ctg ggc agg cac aga gcc gag acc cag gag cag cag agc cga   148
Leu His Leu Gly Arg His Arg Ala Glu Thr Gln Glu Gln Gln Ser Arg
         30                  35                  40

tcc aag agg ggc acc gag gat gag gag gcc aag ggc gtg cag cag tat 196
Ser Lys Arg Gly Thr Glu Asp Glu Glu Ala Lys Gly Val Gln Gln Tyr
    45                  50                  55

gtg cct gag gag tgg gcg gag tac ccc cgg ccc att cac cct gct ggc 244
Val Pro Glu Glu Trp Ala Glu Tyr Pro Arg Pro Ile His Pro Ala Gly
60                  65                  70                  75

ctg cag cca acc aag ccc ttg gtg gcc acc agc cct aac ccc gac aag 292
Leu Gln Pro Thr Lys Pro Leu Val Ala Thr Ser Pro Asn Pro Asp Lys
                80                  85                  90

gat ggg ggc acc cca gac agt ggg cag gaa ctg agg ggc aat ctg aca 340
Asp Gly Gly Thr Pro Asp Ser Gly Gln Glu Leu Arg Gly Asn Leu Thr
            95                  100                 105

ggg gca cca ggg cag agg cta cag atc cag aac ccc ctg tat ccg gtg 388
Gly Ala Pro Gly Gln Arg Leu Gln Ile Gln Asn Pro Leu Tyr Pro Val
        110                 115                 120

acc gag agc tcc tac agt gcc tat gcc atc atg ctt ctg gcg ctg gtg 436
Thr Glu Ser Ser Tyr Ser Ala Tyr Ala Ile Met Leu Leu Ala Leu Val
    125                 130                 135

gtg ttt gcg gtg ggc att gtg ggc aac ctg tcg gtc atg tgc atc gtg 484
Val Phe Ala Val Gly Ile Val Gly Asn Leu Ser Val Met Cys Ile Val
140                 145                 150                 155

tgg cac agc tac tac ctg aag agc gcc tgg aac tcc atc ctt gcc agc 532
Trp His Ser Tyr Tyr Leu Lys Ser Ala Trp Asn Ser Ile Leu Ala Ser
                160                 165                 170

ctg gcc ctc tgg gat ttt ctg gtc ctc ttt ttc tgc ctc cct att gtc 580
Leu Ala Leu Trp Asp Phe Leu Val Leu Phe Phe Cys Leu Pro Ile Val
            175                 180                 185

atc ttc aac gag atc acc aag cag agg cta ctg ggt gac gtt tct tgt 628
Ile Phe Asn Glu Ile Thr Lys Gln Arg Leu Leu Gly Asp Val Ser Cys
        190                 195                 200

cgt gcc gtg ccc ttc atg gag gtc tcc tct ctg gga gtc acg act ttc 676
Arg Ala Val Pro Phe Met Glu Val Ser Ser Leu Gly Val Thr Thr Phe
    205                 210                 215

agc ctc tgt gcc ctg ggc att gac cgc ttc cac gtg gcc acc agc acc 724
Ser Leu Cys Ala Leu Gly Ile Asp Arg Phe His Val Ala Thr Ser Thr
220                 225                 230                 235

ctg ccc aag gtg agg ccc atc gag cgg tgc caa tcc atc ctg gcc aag 772

Leu Pro Lys Val Arg Pro Ile Glu Arg Cys Gln Ser Ile Leu Ala Lys
                240                 245                 250

ttg gct gtc atc tgg gtg ggc tcc atg acg ctg gct gtg cct gag ctc     820
Leu Ala Val Ile Trp Val Gly Ser Met Thr Leu Ala Val Pro Glu Leu
            255                 260                 265

ctg ctg tgg cag ctg gca cag gag cct gcc ccc acc atg ggc acc ctg     868
Leu Leu Trp Gln Leu Ala Gln Glu Pro Ala Pro Thr Met Gly Thr Leu
        270                 275                 280

gac tca tgc atc atg aaa ccc tca gcc agc ctg ccc gag tcc ctg tat     916
Asp Ser Cys Ile Met Lys Pro Ser Ala Ser Leu Pro Glu Ser Leu Tyr
    285                 290                 295

tca ctg gtg atg acc tac cag aac gcc cgc atg tgg tgg tac ttt ggc     964
Ser Leu Val Met Thr Tyr Gln Asn Ala Arg Met Trp Trp Tyr Phe Gly
300                 305                 310                 315

tgc tac ttc tgc ctg ccc atc ctc ttc aca gtc acc tgc cag ctg gtg     1012
Cys Tyr Phe Cys Leu Pro Ile Leu Phe Thr Val Thr Cys Gln Leu Val
                320                 325                 330

aca tgg cgg gtg cga ggc cct cca ggg agg aag tca gag tgc agg gcc     1060
Thr Trp Arg Val Arg Gly Pro Pro Gly Arg Lys Ser Glu Cys Arg Ala
            335                 340                 345

agc aag cac gag cag tgt gag agc cag ctc aac agc acc gtg gtg ggc     1108
Ser Lys His Glu Gln Cys Glu Ser Gln Leu Asn Ser Thr Val Val Gly
        350                 355                 360

ctg acc gtg gtc tac gcc ttc tgc acc ctc cca gag aac gtc tgc aac     1156
Leu Thr Val Val Tyr Ala Phe Cys Thr Leu Pro Glu Asn Val Cys Asn
    365                 370                 375

atc gtg gtg gcc tac ctc tcc acc gag ctg acc cgc cag acc ctg gac     1204
Ile Val Val Ala Tyr Leu Ser Thr Glu Leu Thr Arg Gln Thr Leu Asp
380                 385                 390                 395

ctc ctg ggc ctc atc aac cag ttc tcc acc ttc ttc aag ggc gcc atc     1252
Leu Leu Gly Leu Ile Asn Gln Phe Ser Thr Phe Phe Lys Gly Ala Ile
                400                 405                 410

acc cca gtg ctg ctc ctt tgc atc tgc agg ccg ctg ggc cag gcc ttc     1300
Thr Pro Val Leu Leu Leu Cys Ile Cys Arg Pro Leu Gly Gln Ala Phe
            415                 420                 425

ctg gac tgc tgc tgc tgc tgc tgc tgt gag gag tgc ggc ggg gct tcg     1348
Leu Asp Cys Cys Cys Cys Cys Cys Cys Glu Glu Cys Gly Gly Ala Ser

430                    435                    440

gag gcc tct gct gcc aat ggg tcg gac aac aag ctc aag acc gag gtg    1396
Glu Ala Ser Ala Ala Asn Gly Ser Asp Asn Lys Leu Lys Thr Glu Val
    445                    450                    455

tcc tct tcc atc tac ttc cac aag ccc agg gag tca ccc cca ctc ctg    1444
Ser Ser Ser Ile Tyr Phe His Lys Pro Arg Glu Ser Pro Pro Leu Leu
460                    465                    470                    475

ccc ctg ggc aca cct tgc tgaggcccca gtaggggtgg ggagggaggg           1492
Pro Leu Gly Thr Pro Cys
                480

agaggccgcc acccccgccg gtgtctgctg ttctttcccc ataggtcttg ctttgttgcc 1552

tgtcttgctg tctagggatg gacttggttc ctcttgtcaa ggtttgggaa tgtcaaagcc 1612

ccctccccac acagggcctt tcctgtccct tgtggggcct tccaaccctg tcctttccac 1672

tggtgggcgg tgatgcttct aggtccttag aactgcccag aaactctgag tcccagcagc 1732

tgggagccag aactttgcct gccctccctt ggttccagtc tctcttctct ctctctgcct 1792

tggaacctga ccatacttta gttgtgccct tcccaggcat catcctccta ccaccaacct 1852

ggggccccat cttggaatgg gggctccttg gggccagccc agtgtggctc accacactct 1912

tctttttttt tttttttttt gagatggagt cttgctctgt tgcccaggct ggagtacatt 1972

tgcctgatgt cagctccctg caacctccgc ctcctgggtt caagcgattc tcctgcctca 2032

gcctcctgag tagctgggat tacaggtgtg caccaacaca cccggctaat tttgtatttt 2092

gtagaagagg cggggtttca ccatgttggc caggctggtg ttgaactcct gacctcaagt 2152

gatctgcctg ccttggcctc ccaaagtgct gggattacag gtgtgagctg ccacgcccag 2212

ccccagtaca ctcttctctg gaccagatct gtcccagtcc tggatgcctc ctcctactgg 2272

tgccttcttt tcttccagag gatttctctc tccttcctcc tcctttcttt gggatccctg 2332

ggttgccctg tcccaacctc cttgttaggt gctttcccat aggaggccct tcttgagaaa 2392

caataaacta ggtagaact                                               2411

<210> 2
<211> 481

<212> PRT
<213> Homo sapiens

<400> 2
Met Arg Trp Leu Trp Pro Leu Ala Val Ser Leu Ala Val Ile Leu Ala
1               5                   10                  15

Val Gly Leu Ser Arg Val Ser Gly Gly Ala Pro Leu His Leu Gly Arg
            20                  25                  30

His Arg Ala Glu Thr Gln Glu Gln Gln Ser Arg Ser Lys Arg Gly Thr
        35                  40                  45

Glu Asp Glu Glu Ala Lys Gly Val Gln Gln Tyr Val Pro Glu Glu Trp
    50                  55                  60

Ala Glu Tyr Pro Arg Pro Ile His Pro Ala Gly Leu Gln Pro Thr Lys
65                  70                  75                  80

Pro Leu Val Ala Thr Ser Pro Asn Pro Asp Lys Asp Gly Gly Thr Pro
                85                  90                  95

Asp Ser Gly Gln Glu Leu Arg Gly Asn Leu Thr Gly Ala Pro Gly Gln
            100                 105                 110

Arg Leu Gln Ile Gln Asn Pro Leu Tyr Pro Val Thr Glu Ser Ser Tyr
        115                 120                 125

Ser Ala Tyr Ala Ile Met Leu Leu Ala Leu Val Val Phe Ala Val Gly
    130                 135                 140

Ile Val Gly Asn Leu Ser Val Met Cys Ile Val Trp His Ser Tyr Tyr
145                 150                 155                 160

Leu Lys Ser Ala Trp Asn Ser Ile Leu Ala Ser Leu Ala Leu Trp Asp
                165                 170                 175

Phe Leu Val Leu Phe Phe Cys Leu Pro Ile Val Ile Phe Asn Glu Ile
            180                 185                 190

Thr Lys Gln Arg Leu Leu Gly Asp Val Ser Cys Arg Ala Val Pro Phe
        195                 200                 205

Met Glu Val Ser Ser Leu Gly Val Thr Thr Phe Ser Leu Cys Ala Leu
    210                 215                 220

Gly Ile Asp Arg Phe His Val Ala Thr Ser Thr Leu Pro Lys Val Arg
225                 230                 235                 240

Pro Ile Glu Arg Cys Gln Ser Ile Leu Ala Lys Leu Ala Val Ile Trp
                245                 250                 255

Val Gly Ser Met Thr Leu Ala Val Pro Glu Leu Leu Leu Trp Gln Leu
            260                 265                 270

Ala Gln Glu Pro Ala Pro Thr Met Gly Thr Leu Asp Ser Cys Ile Met
        275                 280                 285

Lys Pro Ser Ala Ser Leu Pro Glu Ser Leu Tyr Ser Leu Val Met Thr
    290                 295                 300

Tyr Gln Asn Ala Arg Met Trp Trp Tyr Phe Gly Cys Tyr Phe Cys Leu
305                 310                 315                 320

Pro Ile Leu Phe Thr Val Thr Cys Gln Leu Val Thr Trp Arg Val Arg
                325                 330                 335

Gly Pro Pro Gly Arg Lys Ser Glu Cys Arg Ala Ser Lys His Glu Gln
            340                 345                 350

Cys Glu Ser Gln Leu Asn Ser Thr Val Val Gly Leu Thr Val Val Tyr
        355                 360                 365

Ala Phe Cys Thr Leu Pro Glu Asn Val Cys Asn Ile Val Val Ala Tyr
    370                 375                 380

Leu Ser Thr Glu Leu Thr Arg Gln Thr Leu Asp Leu Leu Gly Leu Ile
385                 390                 395                 400

Asn Gln Phe Ser Thr Phe Phe Lys Gly Ala Ile Thr Pro Val Leu Leu
                405                 410                 415

Leu Cys Ile Cys Arg Pro Leu Gly Gln Ala Phe Leu Asp Cys Cys Cys
            420                 425                 430

Cys Cys Cys Cys Glu Glu Cys Gly Gly Ala Ser Glu Ala Ser Ala Ala
        435                 440                 445

Asn Gly Ser Asp Asn Lys Leu Lys Thr Glu Val Ser Ser Ser Ile Tyr
    450                 455                 460

Phe His Lys Pro Arg Glu Ser Pro Pro Leu Leu Pro Leu Gly Thr Pro
465                 470                 475                 480

Cys

<210> 3
<211> 26
<212> DNA
<213> Homo sapiens

<400> 3
ctcgggaagc gcgccattgt gttggt 26

<210> 4
<211> 26
<212> DNA
<213> Homo sapiens

<400> 4
gagcccaccc agatgacagc caactt 26

<210> 5
<211> 26
<212> DNA
<213> Homo sapiens

<400> 5
tgaagggcac ggcacgacaa gaaacg 26